# EUROPEAN PATENT SPECIFICATION

(11) **EP 3 322 454 B1**
(45) Date of publication and mention of the grant of the patent: **10.11.2021**
(21) Application number: 16763311.4
(22) Date of filing: 14.07.2016
(51) Int. Cl.: A61L 27/06, A61L 27/42, A61L 27/58, A61L 27/04, A61L 27/56, A61L 31/02, A61L 31/12, A61L 31/14

(54) **COMPOSITE MATERIAL FOR IMPLANTS, ITS USE AND METHOD OF ITS PRODUCTION**
VERBUNDSTOFFMATERIAL FÜR IMPLANTATE, DESSEN VERWENDUNG UND VERFAHREN ZU DESSEN HERSTELLUNG
MATÉRIAU COMPOSITE POUR IMPLANTS, SON UTILISATION ET SON PROCÉDÉ DE PRODUCTION

(30) Priority: 15.07.2015 HR 20150781; 13.07.2016 SK 500462016
(43) Date of publication of application: 23.05.2018
(73) Proprietor: Ústav Materiálov A Mechaniky Strojov SAV, 845 13 Bratislava (SK)
(72) Inventor: BALOG, Martin, 84107 Bratislava (SK); CATIC, Amir, 10000 Zagreb (HR); KRÍZIK, Peter, 82108 Bratislava (SK); SCHAUPERL, Zdravko, 10000 Zagreb (HR)
(74) Representative: Porubcan, Róbert
(86) International application number: PCT/IB2016/054220
(87) International publication number: WO 2017/009805

(56) References cited:
- WO-A1-2012/124661

## Description

### Field of the invention

The invention relates to a composite metal material based on titanium for the production of implants, particularly dental implants. The biocompatible composite material has a low elastic modulus and density, while it retains good mechanical strength and fatigue endurance. The invention also describes a process by which the composite is fabricated.

### Background of the invention

Titanium (Ti) and Ti alloys are widely applied in biomedicine and in particular for manufacturing of biomedical implants. Ti is biocompatible, non-toxic to humans, inert metal, which is chemically resistant to corrosion in the human body, has high specific strength, is sufficiently ductile and has a low density. Ti implants are designed for permanent presence or permanent function in the human body. Typically, commercially pure Ti Grade 3, 4, and 5, as well as Ti alloy Ti-AI6-V4 and other (Ti-Al6-Nb7, Ti-Al5-Fe2.5, Ti-Al6-Mo2-Cr2, etc.) are utilized. The disadvantage of Ti and Ti alloys is their high Young's modulus of elasticity, that is usually above 100 GPa, which is the value several times higher than that of the human bone. Because of the difference in Young's modulus of the implant and the bone, a phenomenon known as stress shielding occurs. As a consequence of a stress shielding phenomenon the implant transvers significantly higher load compared to the bone, which may lead to atrophy or bone osteoporosis and loosening of the implant. Young's modulus of Ti alloys can be reduced by suitable additives, though which will reduce other mechanical properties too or are totally unsuitable from biomedical perspective. It is desired to attain Young's modulus of the implant as much close to Young's modulus of the bone, which is approximately of 30 GPa depending to the type of bone.

For materials used for implants manufacturing an appropriate surface treatment is required too, providing good integration with the bone and other biological tissues in order to achieve a firm and lasting connection. Surface treatment of implants leads to a change of topography, morphology and chemical composition of surface, and specific surface energy. Open specification DE 10 2009 016 554 A1 discloses in Background of the invention a number of surface treatments of implants or materials used for implants fabrication and highlights the new implant of Ti alloy having a surface layer, which comprises zirconium or zirconium oxides and possibly other elements such as magnesium, sodium. The disadvantage of such arrangement is the limited effect of additives located only on the implant surface, high requirements for cleanliness during implant insertion, and the implant must be fitted on the first attempt; otherwise surface contamination takes place, in addition to complicated coating process. The most common methods of implants coating include sandblasting, etching, plasma spraying, anodizing, placing layers of hydroxyapatite and calcium phosphate. These methods are complex, time consuming, and yield to only the limited effect. Similar disadvantages arise from technological approaches according to the open specifications CN105063422 A, CN105056297 A, B1 KR101435158, KR101439512 B1, which describe Ti alloy with a special surface, which is supposed to bring the implant important biomedical effects, such as antibacterial properties.

Publication WO2012124661 A1 discloses a Ti-Mg biomaterial in which Mg is evenly dispersed within Ti, and which is obtained by spar plasma sintering of a mixture obtained by subjecting titanium powder and magnesium powder to mechanical alloying by ball milling process. The titanium biomaterial with Mg content ranging from 10 to 50 wt.% and TiH2, TiC and MgO impurities has higher Vicker hardness than human bones. Neither, the strength, Young's modulus, elongation and fatigue strength, which are crucial properties for a bone reinforcing material or implant material subjected to loading under cyclic conditions, are declared.

A new, currently unknown, material is desired, which could ensure throughout its entire cross-section, not only within its modified surface, required bio-medical effects, which would have suitable Young's modulus, and in the meantime it would maintain high mechanical loading capacity and its manufacturing would be technically feasible.

### Summary of the invention

The invention is defined in claims 1 to 12. The above described disadvantages are substantially eliminated by composite material for implants, which comprises biocompatible Ti or biocompatible Ti alloy as a permanent component (i.e., matrix) and the biodegradable component, which is intended to at least partial disposal in vivo in contact with the human tissue, wherein the composite material is the product of powder metallurgy, and wherein the Ti or Ti alloy forms a bearing permanent structure of the material, according to the present invention, which is characterized in that the material is prepared from a powder mixture of Ti or Ti alloy particles and particles of biodegradable component, which is after fabrication homogenously dispersed evenly throughout materials volume. Biodegradable component has a significantly lower Young's modulus compared to Ti or Ti alloys, typically has Young's modulus lower by at least 40% over Ti or Ti alloys, for example compared with Ti Grade 4 or 5.

Fabrication of the material from the powder mixture of particles of Ti or Ti alloy and particles of biodegradable component will generally include a pressure loading of a mixture, such as extrusion, forging, rolling, hot isostatic pressing (HIP) or similar, wherein the particles of the mixture are consolidated to a bulk body.

The distribution of biodegradable component uniformly throughout the volume of the material means that the biodegradable component is not only on a surface of the material, as the coatings described in the Background of the invention, but is located in an entire cross-section of the material. From the macroscopic point of view this is a uniform distribution, which may be affected by various technological irregularities, however in principle it is a homogeneous distribution of the biodegradable component in the material. At the same time the biodegradable component remains a separate component of the composite, it is not an alloying element in Ti alloy, wherein the biodegradable component forms distinguishable separate phase within the bearing matrix structure of Ti or Ti alloy.

Biodegradable component occupies the volume between 2 and 20% of the total material volume, preferably occupies the volume of 10 to 15% of the total material volume. These volume ratios and the resulting weight ratios according to the density of used biodegradable component provides the necessary mechanical properties, fatigue strength and sufficiently decrease Young's modulus of composite.

Biodegradable component as a phase in a bearing matrix structure is according to the present invention arranged to sections, which are at least partially connected with a surface of the composite. Connection of the sections may be direct, where such sections open directly onto the surface of the material or indirectly, when the section is linked to other adjacent section, which opens directly onto the surface of the material. It is important that at least 15% of biodegradable component is directly or indirectly connected to the surface of the material. As the material is from a macroscopic perspective homogeneous, such connections form also after processing of the material, thus the implant itself will have sections of biodegradable component, which open directly onto the surface of the implant, while at least 15% of biodegradable component will be capable of transportation within the support structure towards the implant surface. This condition is also characterized in that way that at least 2% of the materials surface forms a biodegradable component, which in turn will apply for the machined composite material as well as for the final implant.

Beneficial improvement of the properties of the material for the implant occurs when the biodegradable component in a bearing structure is formed into the filaments oriented in a particular direction, what can be achieved e.g. by using extrusion technology for compaction of the powder mixture to the composite material. Just an extrusion of both composite constituents in particulate powder form yields formation of filaments of biodegradable component. It is achieved due to a lower deformation resistance of biodegradable component compared to deformation resistance of Ti or Ti alloy component, what assures formation of separate filaments of the biodegradable component embedded in a bearing matrix structure of Ti or Ti alloy. Filaments orientation reflects the pressing direction. Although, from the microscopic point of view of the filaments can be of various shapes, lengths and can have a variable cross-section, a connecting line between the start and end points of the filament will have orientation parallel to pressing direction, or it will differ only up to 10°. This will apply to most, usually for more than 90% of fibers.

Biodegradable component represents herein a component that is without negative effects, neutrally or with beneficial medical effects, dissolved in the human body. Such biodegradable component is gradually absorbed or expelled in/from the human body. Magnesium (Mg) proved to be a preferred biodegradable component. Metallic Mg is known as a material for a fabrication of biodegradable biomedical implants (temporary screws, stents, etc.). The temporary connection part made of Mg produces a mechanical join during healing, which gives rise to a reconstruction of the original bone structure, while Mg implant gradually desorbs, is absorbed by the body and physically expires during treatment. Herein, an advantage is taken from a low corrosion resistance of Mg in a contact with body fluids containing water and various organic solvents. Corrosion products are non-toxic and are expelled in a natural way, while in addition Mg has a beneficial effect on the recovery and growth of bone tissue.

Mg in the form of filaments in a Ti matrix bearing structure does not cause problems with the release of large quantities of hydrogen in form of bubbles on a surface of the implant, as is known for the implants made of Mg or Mg alloys. Intensive evolution of hydrogen altogether with possible alkalization of local environment could slow healing process and lead to necrosis of surrounding tissue. These problems, observed in a case of implants made of Mg or Mg alloy, represent a serious problem. However, in the case of Ti - Mg composite Mg desorption is slowed down by the fact that surrounding tissue is in contact only with a small contact surface of Mg filaments. Moreover, Mg dissolvent is slowed down further by native passivation layer of magnesium oxide, present on the surface of feedstock Mg powder, which decorates the surface of Mg filaments in consolidated Mg - Ti composite material.

The present invention is characterized by merging two mutually insoluble metals of different characteristics to the composite, wherein the components are present in separate phases distinguishable from each other, while fibrous state of the biodegradable component embedded in the bearing permanent structure of Ti or Ti alloy provides a significant synergistic effect, which enhances the properties of the composite material. Biodegradable component is not soluble in the crystal structure, but forms a separate phase, whereas the filaments have a cross-sectional dimension of less than 500 µm.

Ti provides a new implant mechanical strength and endurance, Mg lowers Young's modulus and as it desorbs it creates cavities suitable for tissue integration. Generally, the produced composite material is non-porous, respectively with a porosity of the order in units of %, typically up to 1%, due to the nature of powder metallurgy approach used, though it is true, that the pores are not intentionally formed in as-fabricated composite material. The material adopts porous character in vivo, where the biodegradable component is gradually released. Desorption of the biodegradable components contributes to reducing Young's modulus of the composite and in the meantime formed pores increase contact surface area between the implant and the adjacent tissue, thereby improving the mechanical compatibility and load transfer between the implant and the bone. The filament shape of the biodegradable component provides that it is desorbed not only in the zone of a direct contact with living tissue, but gradually also in the volume of the implant. It is therefore important the filament shape of the biodegradable component, for the reason that decomposition products can be transported from the inside volume of the composite. Random individual clusters of biodegradable components which would not be connected to the surface of the implant would remain permanently closed within the implant. Therefore, it is appropriate that at least 15% of a biodegradable component volume is directly or indirectly by mutual contact connected to the implant material. This means that at least 15% of biodegradable material component is in the filaments, which form the channels connected to the surface of the material. At least 15% of the biodegradable component is capable of transportation through channels towards the material surface, i.e. the surface of the implant. Biodegradable component comprises at least 2% of the surface of the implant; these areas create input channels to the material volume.

The filament shaped and textured arrangement of the biodegradable component throughout a volume of the composite material enables to manufacture implants of any shape. Regardless of the implant's shape manufactured from the composite material a part of the filaments ends up onto the surface of the implant, and through such contact areas desorption of the biodegradable components starts. Some of the filaments, e.g., such filaments whose go through threads, may extend integrally from one contact surface of the implant to the counter surface of the implant. Channel which forms after desorption of such filament is to have a closed character, which increases the bonding strength.

The material for the implants in this invention refers to a material of the implant or the material of semi-finished implant product, i.e., compacted blank. Composite material in the form of rods will be usually divided and machined into the desired shape, e.g., threads will be machined, but in principle the composite can be fabricated directly in the form of the final implant. Therefore, the term "material" should be understood broadly as a substance for the fabrication, or a semi-finished blank as well as a mass of the final implant.

Mg has Young's modulus of 45 GPa, which is considerably less than that of Ti, which about 110 GPa. Composite material with a Mg filaments has Young's modulus less than Ti itself. Moreover, Young's modulus of the implant decreases further as Mg disrobes.

Young's modulus of the composite material decreases right after a few days the implant is placed into the body. This effect was verified by measuring of Young's modulus using the samples (Ti-12 vol.% Mg) immersed to physiological (Hank's) solution.

| Sample condition | E (GPa) |
|---|---|
| Extruded | 92.1 ± 0.6 |
| After 1 day dilution | 91.8 ± 0.6 |
| After 3 days dilution | 91.4 ± 0.9 |

As an important way of utilization appears to be dental applications. Dental implants require high mechanical strength; teeth are often loaded extremely and locally; load transfer to the jaw has yet cyclical nature. Dental implants have to meet demanding requirements of fatigue performance up to 10⁶ fatigue cycles as defined by the ISO 14801 standard. Ti matrix with biodegradable component or with partially or completely desorbed biodegradable component provides mechanical and fatigue strengths at the level of strengths for Ti grade 3-5.

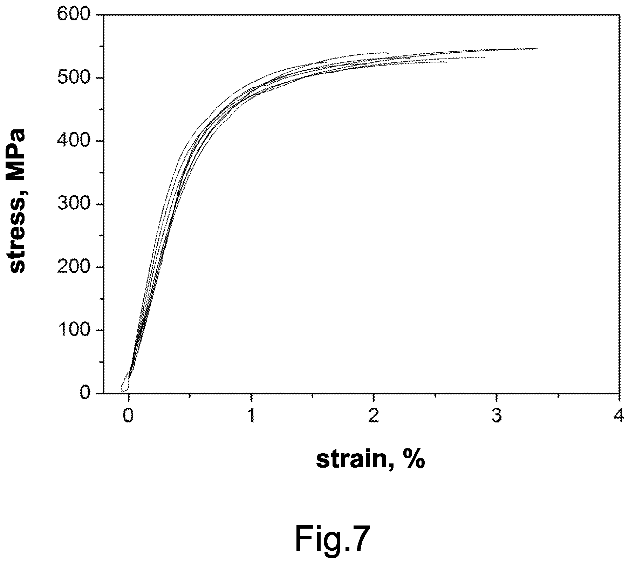

Using the material Ti + 12 vol.% Mg the following values of ultimate tensile strength (UTS), yield strength at 0.2% offset of a strain (YS) and elongation (ε) were measured during tensile tests realized at room temperature (also on Figure 7). For comparison the minimum required values for Ti grade 4 are included too.

| Material | reference | UTS (MPa) | YS (MPa) | ε (%) |
|---|---|---|---|---|
| Ti + 12 vol.% Mg | this invention | 529 ± 7 | 450 ± 12 | 1.4 ± 0.3 |
| Ti grade 4, minimum | ASTM B265 | 550 | 483 | 15 |

The drawbacks mentioned in the Background of the invention are substantially eliminated by the method of making the composite material of an implant; the method comprises providing particles of at least two components, mixing them to homogenous mechanical mixture and subsequent pressing consolidation at a temperature above 300 °C., according to the invention, which comprises that, after mixing particles of Ti or Ti alloy with particles of biodegradable component the mixture is pressed under a pressure of at least 20 MPa and at a temperature up to 640 °C to obtain consolidated composite material. Provided extrusion is used as consolidation method biodegradable particles got elongated into filaments oriented along extrusion direction. Formation of filaments occurs owing to consolidation realized by extrusion, where Ti particles of higher deformation resistance deform particles of biodegradable component of lower deformation resistance. Formation of filament shaped phase of biodegradable component and its morphology depend on the size of the particles, reduction ratio, speed and temperature used during extrusion.

In a preferred configuration, the maximum pressing temperature is 500 °C, at which undesired reactions of Ti powder with ambient atmosphere, e.g., oxidation, nitridation, and with working tools are avoided. Nevertheless, in principle it is possible to increase the pressing temperature up to limit temperature of 640 °C, which is limited by the melting point of Mg (649.85 °C).

In addition to extrusion the other hot working compaction techniques may be used, such as forging, rolling, etc. Extrusion has proven to be technologically simple and feasible, and productive. The reduction ratio used during extrusion compaction may be in the range from 5:1 to 100:1, preferably 16:1 according to the exemplary embodiment.

The powders of components have mean particle size of 5 to 1000 µm and one component of the composite is Ti or Ti alloy, and that component represents 80% to 98% by volume of the material.

Since as-blended powder mixture of the components is homogeneous, consolidation yields essentially homogeneous structure of biodegradable component embedded in Ti or Ti alloy. Ti matrix herein provides a bearing function. As it is apparent from the description of the composite material, the biodegradable component is preferably Mg. Upon extrusion Mg particles combined into filaments that are oriented along pressing direction. The filaments of the biodegradable component in this document refer to fibrous structures, capillaries, channels or elongated bodies of biodegradable component. The powder particles of biodegradable component(s) and Ti or Ti alloy may have prior consolidation shape of granules, short fibres (whiskers) or even fibres.

It appeared that a preferred technological process is when blended powder mixture was cold-isostatically pressed (CIP), by which handling strength of the mixture is achieving. At the same time, the powder precompact can be more easily and securely heated up to the pressing temperature, and then extruded. Precompaction also improves heat transfer and thus homogenizes heating of the mixture to the required extrusion temperature. Low temperature, to which the powder precompact is heated to during extrusion, results in the limited reactivity of Ti or Ti alloy powder with atmospheric gases (oxygen, nitrogen) and with the die.

The composite according to this invention is characterised by good osseointegration potential. The biodegradable component, in particular in the form of Mg, is gradually resorbed into the body, thereby forming a porous structure of the implant, overgrown by living tissue, while Young's modulus reduces, what contributes to reduction of stress shielding. The composite material is fabricated by highly productive method: low temperature extrusion compaction, while the resulting material is not susceptible to contamination, since the pores within the matrix Ti structure are filled with the biodegradable component till the point of implant placement to the human body. The composite according to this invention has high fatigue strength, which enables utilize it for the dental implant, which are subjected to high mechanical loading.

### Description of Drawings

The invention is further described by Figures 1 to 7. Displayed volume ratio of the Ti and Mg as well as particular shape and morphology of the Mg filaments is merely illustrative and cannot be interpreted as narrowing the scope of protection.
In Figure 1 the cross-section of the composite material in a plane perpendicular to the extrusion axis, as viewed in scanning electron microscope, is shown. The black contrast areas in Ti matrix are areas, where Mg filaments open to the surface. The filaments continue down below the surface into volume of the material, as shown in Figure 2.
Figure 2 is the cross-section of the composite material in a plane parallel to the extrusion axis, as viewed in scanning electron microscope. Mg filaments have relatively random shape, but are in general oriented along extrusion axis.
Figure 3 represents a schematic view of the threaded implant and the detail of one thread cross-section with illustration of some filaments biodegradable component.
Figure 4 is the cross-section of the composite material in a plane perpendicular to the extrusion axis, as viewed in scanning electron microscope, after 1 day exposure to physiological (Hank's) solution. The image shows cavities formed as a result of Mg desorption. The absence of Mg is visible on the edges of cavities that were previously filled with Mg as shown in Figure 1. Therefore in Figure 4 there is no included label 2, which corresponds to desorbed biodegradable component.
Figure 5 shows Ti particles prior to the mixing and consolidation. Figure 6 shows Mg particles prior to mixing and consolidation. Both images were obtained by scanning electron microscope.
Figure 7 shows the stress-strain curves obtained from tensile tests carried out at room temperature using the samples of the material according to the invention.

### The exemplary embodiment

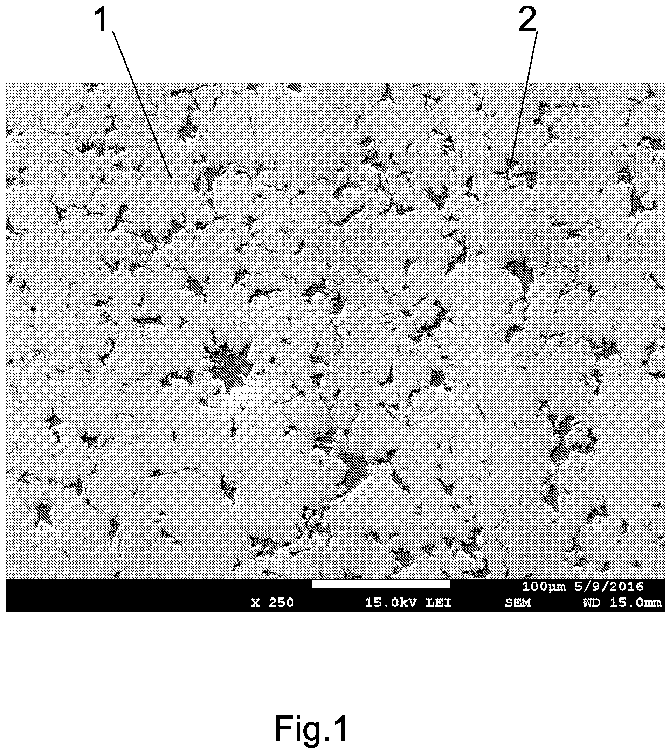

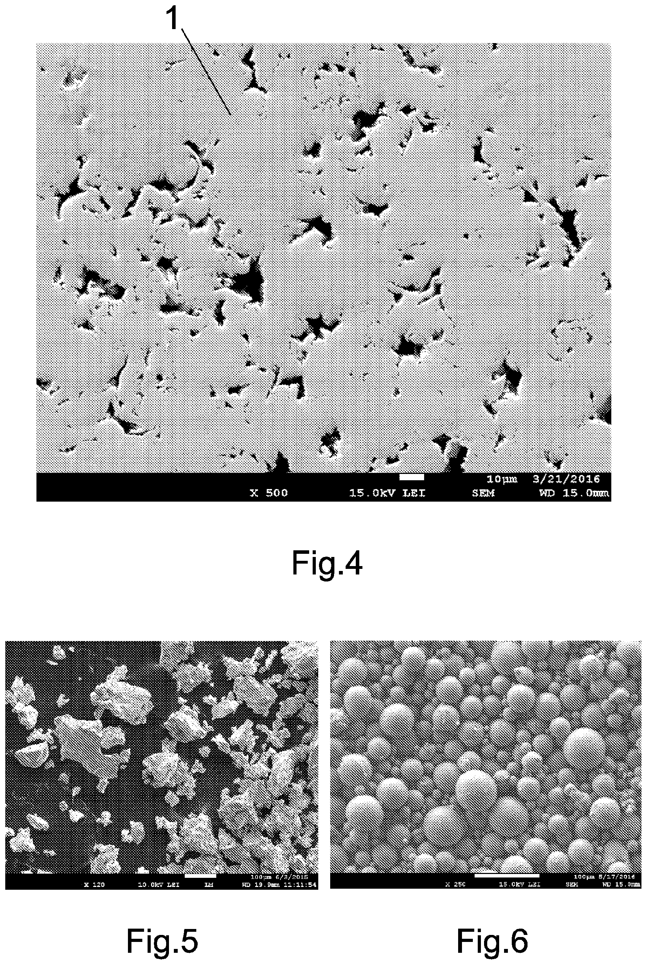

In this example based on the Figures 1 - 7, in order to obtain a high strength of composite material and in the meantime to have the filaments of the biodegradable component of sufficient size (sufficient for osseointegration to proceed) the powder mixture of Ti and 12 vol.% Mg is used. Ti powder is of <99% purity, has mean (median) particle size d₅₀ = 85 µm, what corresponds to a powder fraction <150 µm.Ti powder is blended with Mg powder of <99% purity and with mean (median) particle size d₅₀ = 30 µm. An example of as-received powders materials is shown in Figures 5 and 6.

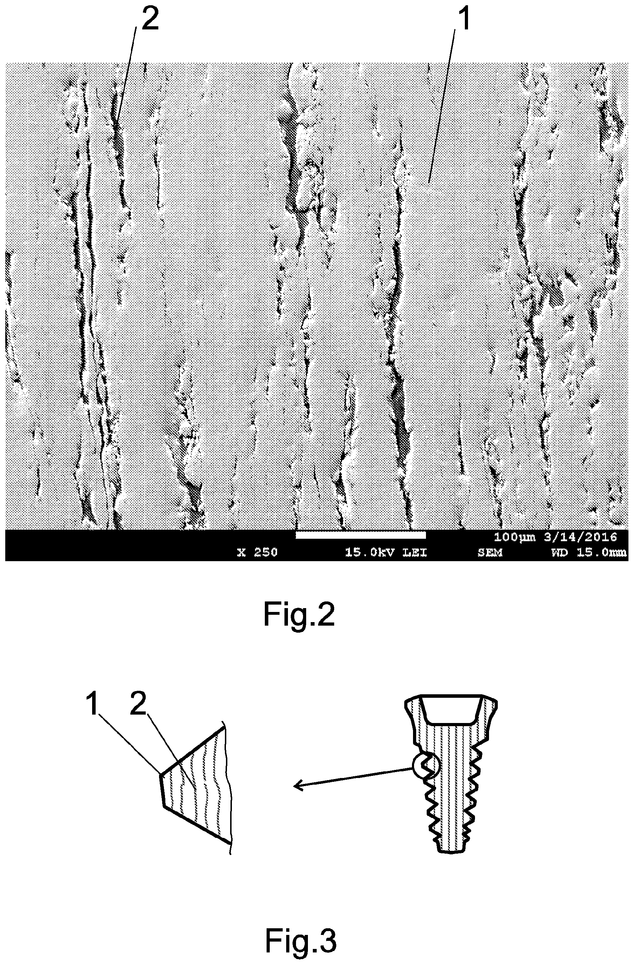

The mixture is homogenized for a period of ∼ 30 min in a blender. The powder mixture is then cold compacted by CIP at 150 MPa. CIPed powder blank is consolidated by direct extrusion at 475 °C at a reduction ratio of 16:1, to a final form of Ti + 12 vol.% Mg composite, in this example to a rod with a diameter of 7.5 mm. After extrusion, the material is spontaneously cooled down to room temperature. The microstructure of as-extruded composite material is shown in Figures 1 and 2 obtained by a scanning electron microscope SEM, JEOL 7500.

The tensile properties of the tensile bars with gauge diameter of 3 mm and gauge length of 21 mm were tested using a Zwick Roell 1474 machine in accordance with ASTM E8 and ASTM E21 standards. Eight samples were tested during tensile test was. Young's modulus was measured by DMA method, by three-point bending test using TA Q800 machine. The samples were extracted longitudinally from the extruded profile, after cutting a surface of the samples was dry polished. The dimensions of the samples were determined with precision of 0.005 mm. Testing was performed at room temperature, at a frequency of 1 Hz, using a preloading force of 0.5 N and a maximum load of 3.5 N for 4 min. Young's modulus was determined using two samples, where each sample was measured four times on each side. The measurement error was less than 1%.

Fatigue testing of dental implants fabricated from the composite material was performed in accordance with the ISO 14801 standard for dental implants. Implants were mounted in a PMMA holder at an angle of 60° with the horizontal. For testing Walter + Bai AG, LFV HH-50 machine was used.

The samples from as-extruded composite were immersed to Hank's solution at 37 °C. After 1 day dilution in Hank's solution the specimens were subsequently immersed to 10 g.l⁻¹ CrO₃ solution for one minute to clean the corrosion product. The same procedure was conducted after additional 2 days immersion in Hank's solution, resulting in an overall immersion time of 3 days.

In this example the implant material has a low density of 4.12 ± 0.02 g.cm⁻³. Determined density is approximately 9% lower compared to the Ti grade 4 reference material. Further decrease of composite's density is expected due to depletion of Mg upon implantation. The apparent density of as-extruded composite material is 98.8 ± 0.44% of the theoretical density. The porosity of ∼1.2% is fully acceptable assuming that material is manufactured via powder metallurgy approach. The porosity will escalate later due to Mg dilution upon implantation.

Mg phase in as-extruded composite is noticeably oriented into an extrusion direction and is homogeneously dispersed in Ti matrix, with no formation of distinct conglomerates confirmed. Detailed investigation of Ti - Mg interfaces proved that those interfaces are generally free of porosity and voids.

Tensile tests confirmed very good mechanical properties and their good reproducibility, as shown in Figure 7. The average values of ultima tensile strength, yield strength and elongation are given in the Summary of the invention. In general, Mg phase does not contribute to a load transfer in the composite. Composite material according to the invention achieves very good strengths (UTS, YS) which are close to the required minimum values for Ti grade 4, the reference material for dental implants, as defined by the ASTM B265 standard.

Young's modulus of as-extruded composite material is 92.1 ± 0.6 GPa, which is ∼ 12% less than that of Ti grade 4. Young's modulus further reduces upon exposure of the composite material in the Hank's solution for 3 days and desorption of Mg filaments from the surface and volume of the composite down to 91.4 ± 0.9 GPa.

The implants samples fabricated from the composite material endured dynamic testing without fracture for a period of 5·10⁶ cycles and sinusoidal loading of 30-300 N. Obtained values of fatigue strength are on the level of those implants fabricated from Ti grade 3. No micro-cracking was detected during subsequent surface analysis of the tested samples.

### Industrial applicability

Industrial applicability is obvious. According to the invention, the composite material can be industrially and repeatedly fabricated and use, particularly for fabrication of dental implants with high cyclic strength and excellent biocompatibility with living tissue.

### List of reference labels

1 - titanium
2 - biodegradable component

## Claims

1. A composite material for the implants comprising biocompatible titanium (1) or biocompatible titanium alloy and comprising a biodegradable component (2), which is intended for at least partial desorption in vivo in contact with a human tissue,
wherein a material is a product of a powder consolidation,
wherein titanium (1) or titanium alloy forms a bearing structure,
wherein the material is fabricated by a consolidation of a powder mixture of titanium (1) or titanium alloy particles and particles of the biodegradable component (2),
and wherein the biodegradable component (2) is as a phase dispersed homogenously throughout a volume of the material
and the biodegradable component (2) occupies from 2 to 20 volumic % of the material,
**is characterized by the fact, that**
the biodegradable component (2) is arranged in a bearing structure by an extrusion to sections, of which at least 15% is directly or indirectly connected to a surface of the material
and the sections of the biodegradable component (2) are in the form of filaments oriented along the extrusion direction.

2. The composite material for the implants as claimed in claim **1 is characterized by the fact that** the biodegradable component (2) is magnesium.

3. The composite material for the implants as claimed in claim 2 **is characterized by the fact that** magnesium occupies from 11 to 13 volumic % of the material.

4. The composite material for the implants as claimed in any of claims 1 to 3 **is characterized by the fact that** the implant is a dental implant, preferably as an artificial tooth's root intended to be implanted into a bone of a jaw or a mandible.

5. A process for manufacturing of a composite implant, comprising steps of a providing powders of at least two components, a mechanical blending to a homogeneous mixture, and a subsequent pressing at a temperature above 300 °C., wherein first component is represented by titanium (1) or titanium alloy, and that first component occupies 80% to 98 volumic % of a material, a second component is formed of a biodegradable component (2),
**is characterized by the fact that**
titanium or titanium alloy powder has an mean (median) particle size d₅₀ from 5 to 1000 µm,
after blending, the powder of titanium or titanium alloy with the powder of biodegradable component (2) are pressed together under a pressure of at least 20 MPa and at a temperature up to 640 °C, by which they are consolidated to a common mass,
and the powder mixture is consolidated by an extrusion, during which the biodegradable component (2) is formed into filaments oriented along the extrusion direction.

6. The process for manufacturing of the composite implant as claimed in claim 5 **is characterized by the fact that** the biodegradable component (2) is magnesium.

7. The process for manufacturing of the composite implant as claimed in claim 5 or 6 **is characterized by the fact that** the reduction ratio during extrusion is 5:1 to 100:1, preferably 16:1.

8. The process for manufacturing of the composite implant as claimed in any of claims 5 to 7 **is characterized by the fact that** the powder mixture is pressed at a temperature up to 500 °C.

9. The process for manufacturing of the composite implant as claimed in any of claims 5 to 8 **is characterized by the fact that** the powder mixture is cold compacted by a cold isostatic pressing (CIP) in order to achieve a handling strength, and a resulting precompact is then heated and pressed.

10. The process for manufacturing of the composite implant as claimed in any of claims 5 to 9 **is characterized by the fact that** after cooling the material is machined to a shape and dimensions of the implant.

11. The process for manufacturing of the composite implant as claimed in any of claims 5 to 10 **is characterized by the fact that** titanium powder is of a purity of at least 99% and a fraction smaller than 150 µm.

12. The process for manufacturing of the composite implant as claimed in any of claims 5 to 11 **is characterized by the fact that** magnesium powder is of a purity of at least 99%, and has a mean size of less than 50 µm.

## Patentansprüche

1. Verbundstoffmaterial für Implantate, umfassend biokompatibles Titan (1) oder eine biokompatible Titanlegierung und umfassend eine biologisch abbaubare Komponente (2), die für einen zumindest teilweisen Abbau in vivo in Kontakt mit menschlichem Gewebe bestimmt ist;
wobei das Material ein Pulvermetallurgieprodukt ist,
und wobei das Titan (1) oder die Titanlegierung eine Tragstruktur bildet,
wobei das Material durch Verdichtung eines Gemisches von Pulverteilchen von Titan (1) oder von Titanlegierung und Pulverteilchen einer biologisch abbaubaren Komponente (2);
und wobei die biologisch abbaubare Komponente (2) als Phase gleichmäßig über das Gesamtvolumen des Materials verteilt ist,
und die biologisch abbaubare Komponente (2) ein Volumen im Umfang von 2 bis 20 % des Gesamtvolumens des Materials einnimmt,
**dadurch gekennzeichnet, dass**
die biologisch abbaubare Komponente (2) in der Tragstruktur durch Extrusion zu Abschnitt angeordnet ist, von denen mindestens 15 % direkt oder indirekt mit der Oberfläche des Materials miteinander verbunden sind
und der Abschnitt der biologisch abbaubaren Komponente (2) die Form von in der Extrusionsrichtung ausgerichteten Fasern haben.

2. Verbundstoffmaterial für Implantate nach Anspruch 1, **dadurch gekennzeichnet, dass** die biologisch abbaubare Komponente (2) Magnesium ist.

3. Verbundstoffmaterial für Implantate nach Anspruch 2, **dadurch gekennzeichnet, dass** das Magnesium 11 bis 13 % des Volumens des Materials einnimmt.

4. Verbundstoffmaterial für Implantate nach einem der Ansprüche 1 bis 3, **dadurch gekennzeichnet, dass** das Implantat ein Zahnimplantat ist, vorzugsweise als künstliche Zahnwurzel, die zum Einsetzen in den Knochen des Ober- oder Unterkiefers bestimmt ist.

5. Verfahren zur Herstellung eines Verbundstoffmaterials für Implantate, das die Herstellung von Pulvern aus mindestens zwei Komponenten, deren Mischen zu einer homogenen mechanischen Mischung und das anschließende Pressen bei einer Temperatur von mehr als 300°C umfasst, wobei die erste Komponente Titan (1) oder eine Titanlegierung ist und diese erste Komponente 80 % bis 98 % des Volumens des Materials ausmacht und die zweite Komponente aus einer biologisch abbaubaren Komponente (2) besteht,
**dadurch gekennzeichnet, dass**
das Titan- oder Titanlegierungspulver eine mittlere (Median-) d₅₀-Partikelgröße von 5 bis 1000 µm aufweist,
nach dem Mischen das Titan- oder Titanlegierungspulver mit dem Pulver der biologisch abbaubaren Komponente (2) zusammen unter einem Druck von mindestens 20 MPa bei einer Temperatur von bis zu 640°C gepresst werden, wodurch sie zu einer gemeinsamen Masse verdichtet werden,
und das Pulvergemisch wird während der Extrusion verdichtet, während der die biologisch abbaubare Komponente (2) zu Fasern mit Ausrichtung in Extrusionsrichtung geformt wird.

6. Verfahren zur Herstellung eines Verbundstoffmaterials für Implantate nach Anspruch 5, **dadurch gekennzeichnet, dass** die biologisch abbaubare Komponente (2) Magnesium ist.

7. Verfahren zur Herstellung eines Verbundstoffmaterials für Implantate nach Anspruch 5 oder 6, **dadurch gekennzeichnet, dass** das Reduktionsverhältnis bei der Extrusion 5:1 bis 100:1, vorzugsweise 16:1 beträgt.

8. Verfahren zur Herstellung eines Verbundstoffmaterials für Implantate nach einem der Ansprüche 5 bis 7, d a **durch gekennzeichnet**, dass das Pulvergemisch bei einer Temperatur von bis zu 500 ° C gepresst wird.

9. Verfahren zur Herstellung eines Verbundstoffmaterials für Implantate nach einem der Ansprüche 5 bis 8, **dadurch gekennzeichnet, dass** das gemischte Pulvergemisch durch kaltisostatisches Pressen (CIP) kaltverdichtet ist, um eine Handhabungsfestigkeit zu erreichen, und der resultierende Pressrohling anschließend erwärmt und gepresst wird.

10. Verfahren zur Herstellung eines Verbundstoffmaterials für Implantate nach einem der Ansprüche 5 bis 9, **dadurch gekennzeichnet, dass** das Material nach dem Abkühlen in die Form und die Abmessungen des Implantats zerspant wird.

11. Verfahren zur Herstellung eines Verbundstoffmaterials für Implantate nach einem der Ansprüche 5 bis 10, d a **durch gekennzeichnet**, dass das Titanpulver eine Reinheit von mindestens 99 % und eine Fraktion von weniger als 150 µm aufweist.

12. Verfahren zur Herstellung eines Verbundstoffmaterials für Implantate nach einem der Ansprüche 5 bis 11, d a **durch gekennzeichnet**, dass das Magnesiumpulver eine Reinheit von mindestens 99 % und eine mittlere Korngröße von weniger als 50 µm aufweist.

## Revendications

1. Un matériau composite pour implants comprenant du titane biocompatible (1) ou un alliage de titane biocompatible et comprenant un composant biodégradable (2) destiné à une dégradation au moins partielle in vivo en avec du tissu humains où le matériau est produit de la métallurgie des poudres,
et dans lequel le titane (1) ou l'alliage de titane forme structure porteuse
où le matériau est fabriqué par consolidation du mélange des particules de poudre de titane (1) ou d'alliage de titane et des particules de poudre du composant biodégradable (2),
et où le composant biodégradable (2) est réparti sous forme de phase de manière homogène dans tout le volume du matériau,
et le composant biodégradable (2) occupe un volume de 2 à 20 % du volume total du matériau,
**caractérisé par le fait que**
le composant biodégradable (2) est arrangé dans la structure porteuse par extrusion en structures dont au moins 15 % sont directement ou indirectement reliées les unes aux autres avec la surface du matériau
et ces structures du composant biodégradable (2) sont sous forme de fibres orientées dans le sens de l'extrusion

2. Le matériau composite pour implants selon la revendication 1 **caractérisé par le fait que** le composant biodégradable (2) est magnésium

3. Le matériau composite pour implants selon la revendication 2 **caractérisé par le fait que** magnésium occupe de 11 à 13% du volume du matériau.

4. Le matériau composite pour implants selon quelleconque des revendications 1 à 3, **caractérisé par le fait que** l'implant est implant dentaire, de préférence sous forme d'un racine dentaire artificielle destinée à être insérée dans l'os des mâchoires.

5. Un procédé d'une fabrication du matériau composite pour implants, comprenant une préparation des poudres composés d'au moins de deux composants, leur mélange en un mélange mécanique homogène puis pressage à une température supérieure à 300°C, le premier composant étant du titane (1) ou d'un alliage titane et ce premier composant représente 80 à 98 % du volume du matériau, le deuxième composant est en composant biodégradable (2)
**caractérisé par le fait que**
une poudre de titane ou d'alliage de titane a une granulométrie moyenne (médiane) d₅₀ de 5 à 1000 µm,
après mélanger de la poudre de titane ou d'alliage de titane avec de la poudre du composant biodégradable (2) sont pressées ensemble sous une pression d'au moins 20 MPa à une température jusqu'à 640°C, les consolidant ainsi en une masse commune,
et le mélange de poudre est consolidé par extrusion, pendant laquelle le composant biodégradable (2) est transformé en fibres orientés dans le sens de l'extrusion

6. Le procédé de la fabrication du matériau composite pour implants selon la revendication 5 **caractérisé par le fait que** le composant biodégradable (2) est magnésium.

7. Le procédé de la fabrication du matériau composite pour implants selon la revendication 5 ou 6 **caractérisé par le fait q**'un rapport de réduction pendant l'extrusion est de 5:1 à 100:1, de préférence 16: 1.

8. Le procédé de la fabrication du matériau composite pour implants selon quelleconque des revendications 5 à 7 **caractérisé par le fait q u e** le mélange de poudre est pressé à une température jusqu'à 500°C.

9. Le procédé de la fabrication du matériau composite pour implants selon quelleconque des revendications 5 à 8 **caractérisé par le fait que** le mélange préparé de poudres est pré-pressé à froid isostatiquement (CIP) pour obtenir une résistance à la manipulation et une prépièce résultante est ensuite chauffée et pressée.

10. Le procédé de la fabrication du matériau composite pour implants selon quelleconque des revendications 5 à 9 **caractérisé par le fait** q u ' après refroidissement, le matériau est usiné par enlèvement de copeaux en forme et dimensions de l'implant.

11. Le procédé de la fabrication du matériau composite pour implants selon quelleconque des revendications 5 à 10 **caractérisé par le fait que** la poudre de titane a une pureté d'au moins 99 % et a une fraction inférieure à 150 µm.

12. Le procédé de la fabrication du matériau composite pour implants selon quelleconque des revendications 5 à 11 **caractérisé par le fait que** la poudre de magnésium a la pureté d'au moins 99% et la granulométrie moyenne inférieure à 50 µm.
